# EUROPEAN PATENT SPECIFICATION

(11) **EP 4 240 277 B1**
(45) Date of publication and mention of the grant of the patent: **20.08.2025**
(21) Application number: 21889748.6
(22) Date of filing: 25.10.2021
(51) Int. Cl.: A61C 17/20, A61C 3/03, A61C 17/22, A61N 7/00

(54) **A DEVICE FOR CLEANING TARTAR BY MEANS OF SOUND WAVE**
VORRICHTUNG ZUR REINIGUNG VON ZAHNSTEIN MITTELS SCHALLWELLEN
DISPOSITIF DE NETTOYAGE DE TARTRE AU MOYEN D'ONDES SONORES

(30) Priority: 06.11.2020 TR 202017732
(43) Date of publication of application: 13.09.2023
(73) Proprietor: T.C. Ankara Universitesi Rektorlugu, Ankara (TR)
(72) Inventor: KILICARSLAN, Mehmet Ali, Cankaya/Ankara (TR)
(74) Representative: Kaya, Erdem
(86) International application number: PCT/TR2021/051092
(87) International publication number: WO 2022/098330

(56) References cited:
- WO-A1-2010/132496
- WO-A1-2016/170520
- WO-A2-2006/119376
- WO-A2-2006/119376
- CN-A- 107 411 837
- DE-A1- 102005 044 074
- US-A- 4 331 422
- US-A1- 2007 149 881
- US-A1- 2008 155 770
- US-A1- 2015 111 169

## Description

### Technical field of the invention

The invention relates to a device for cleaning tartar by means of sound wave that directs sound waves to the tartar and disrupts the structure of the tartars and allows them to be removed from the mouth.

### State of the art of the invention

The proliferation of bacteria in the human mouth with the contents of the mouth and saliva during feeding causes the teeth in the mouth to be covered with a sticky and colorless (or pale yellow) film. This plate layer causes cavities in the tooth enamel due to its acidic structure, and food residues that cannot be cleaned due to this cavity cause caries when it is not cleaned regularly.

Meanwhile, the plate layer around the tooth causes the formation of calculus/tartar structures by hardening as a result of the precipitation of the minerals in them together with the saliva and gingival crevicular fluid when it is not cleaned regularly. These structures are located between the gums and teeth, causing deterioration of oral hygiene and triggering various oral health problems.

These tartar structures are the main cause of inflammatory disease characterized by redness of the gum, edema, bleeding and resorption in the jawbone (alveolar bone), which is called gingivitis or periodontitis, and consequently tooth loss. The initial and most important step in the treatment of these diseases is the mechanical removal of the tartar, which is the cause of the diseases, from the teeth and periodontal tissues.

The most traditional method used for this purpose is the use of hand tools called scaler or curette. The dentist scrapes away the tartar accumulated around the tooth with hand-wrist force with these hand tools resembling a hook. Tartars, which take form according to where they are formed in the mouth and pass tightly between the gums and teeth over time, tend to remain constant in the area where they are located. Therefore, it is necessary to apply high force in the patient's mouth and to intervene in the gums during the application of the said method. It causes the patient's well-being to decrease although the patient feels high pain and pain for a while for this reason, even though it is an effective method. It also contains ergonomic problems that may occur during the application of the art.

A manipulation requiring hand and wrist movement is applied during the use of hand tools called scaler or curette. Therefore, this method is a time-consuming technique that can cause ergonomic problems for both physicians.

Electrical devices have been developed in recent years that perform vibration at a frequency between 20-45 kHz with scaler-like tips attached to the tip in order to facilitate the forced manipulation of the scalers. The said devices use piezoelectric or magnetic shrinkage methods that convert electrical energy into mechanical energy.

In devices operated with the piezoelectric method, these materials are transformed by applying electricity to quartz crystals or customized ceramics. Volumetric changes that occur as a result of shape change are converted into mechanical movement with the help of various assemblies. The frequency of the mechanical movement obtained as a result of the shape change and thus the frequency of the vibration can be adjusted by changing the current applied on the material performing the shape change in the devices operating with this phenomenon. Similarly, the frequency of the vibration can be adjusted with the changes in the electric field in the devices operating with the magnetic shrinkage method.

Cavitron Devices are mechanically contacted on the tooth and tartar, allowing the vibration to be transferred to the plaque and tartar. Shock waves caused by vibration cause weakening in the bonds of tartar structures with the tooth. It is ensured that they are divided into smaller pieces with the deterioration of the structure of the tartar.

The deterioration of the tartar structure allows it to be separated from the tooth and cleaned. Meanwhile, there is a liquid channel that targets the tip point where vibration is provided in cavitron devices. The main purpose of these fluid channels is to permanently provide fluid flow at the tip point of the cavitron device in contact with the tooth and the tooth, thereby eliminating the high heat generated during operation of the device. This fluid channel also prevents the heating of the cavitron tips, which vibrate on the working surface and exert force on the tooth. These fluids used to cool the cavitron tips pose a very high risk for crossinfection since heat is released during its operation. It also causes problems such as dazzling and pain due to vibration that contacts the tooth. Aerosol-generating devices such as traditional cavitron devices pose a risk for droplet-borne infections. These aerosols can splash pathogenic microorganisms that can be found both in the mouth and throat flora and in the blood to 20-25 times more area than normal speech distance since they are already studied in a hemorrhagic area. Therefore, the presence of aerosols can significantly increase the risk of transmission for many diseases that are easily transmitted by respiration due to droplets such as COVID-19 or that can be transmitted through blood, such as Hepatitis. In addition, the pain in the treatment process reduces the comfort of the treatment for the patient and creates difficulties for the physician to work.

There are some studies on the use of acoustic waves for tartar cleaning when the known state of the art is examined.

The US patent application numbered US4331422A refers to a cavitron device that can actuate the abrasive parts in the liquid transferred to the tooth periphery from a water channel in the tip region with an acoustic energy generator and thus clean the teeth. The liquid transferred from the transfer head to the dental vein is contacted with an acoustic source and the vibration at the ultrasonic wavelength is transferred to the liquid and transported to the tooth and its surroundings within the scope of the invention. The abrasive particles moving in the liquid clean the tartar thanks to this wave carried. There is a possibility of damage to the tooth since the tooth and the tartar are not distinguished in the present invention. In addition, the invention will also cause loss in the tooth material by providing undesirable effects as well as the desired effects since there is no special tip (nozzle) design that will transfer the liquid to the tooth area instead of a part design for the transfer of the liquid.

A tooth cleaning device that breaks down tartar and similar formations by using ultrasonic waves is mentioned in the application of the utility model of the People's Republic of China numbered CN204671301U. Acoustic waves are transmitted to the liquid passing through the water distribution channel, ensuring the movement of the wave in water and thus transforming into a mechanical effect on the tooth surface during the intervention with the device with a tangible body. It is seen that the use of liquid within the scope of the said invention and its damping during the propagation of the wave in the liquid and its reduction of the desired effect are contrary to the working purpose of the invention.

US2008155770A1 discloses power toothbrushes which use acoustic wave action to produce a cleansing effect for the teeth of the user.

### Technical problems that the invention aims to solve

The invention is as defined in the appended claim and relates to a device for cleaning tartar that will enable tartar cleaning to be performed by means of sound waves. There will be no need for liquid use or coolant during use thanks to the invention. Preservation of hygiene conditions without producing aerosol and patient welfare without creating patient sensitivity will be ensured in this way.

No coercive hand or wrist movement is required during the use of the invention. There will be no need for high physical power thanks to the fact that it disrupts and removes the tartar structure without forcing physical contact. Tartar that loses their attachment properties due to the deterioration of the structure can be cleaned quickly. It is also a device that can work in closed areas such as gingival pockets, where the physical tip has difficulty in reaching, as there is no need for forced physical contact.

It is not compulsory to use water for cooling purposes since there is no heating problem during operation. There will be no aerosol dispersion in this way. The patient will be able to work even in the mouth closure reflexes that may occur during treatment since it does not have a sharp and soft tip that damages the tissues such as scaler or cavitron. The patient will not experience pulling and pain in the facial muscles during or after the treatment and the patient's well-being will increase since the patient's mouth opening does not need to be at the largest possible opening.

### Description of the invention

The invention relates to a device for cleaning tartar that disrupts the structure of tartar by directing the sound waves to be emitted from the acoustic wave generator to the tartar and operates without the need for the use of liquid for cooling purposes.

### Description of the drawings

The device for cleaning tartar by means of sound wave realized in order to achieve the stated purposes of the invention is shown in the figures, among these figures;

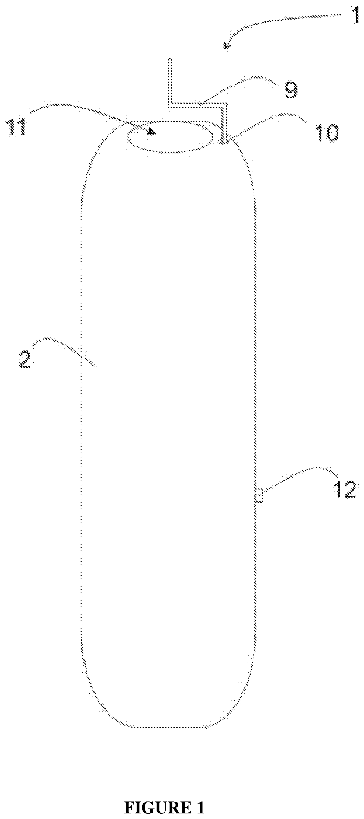

Figure 1 - Schematic representation of the side and outside view of the device for cleaning tartar by means of sound wave.

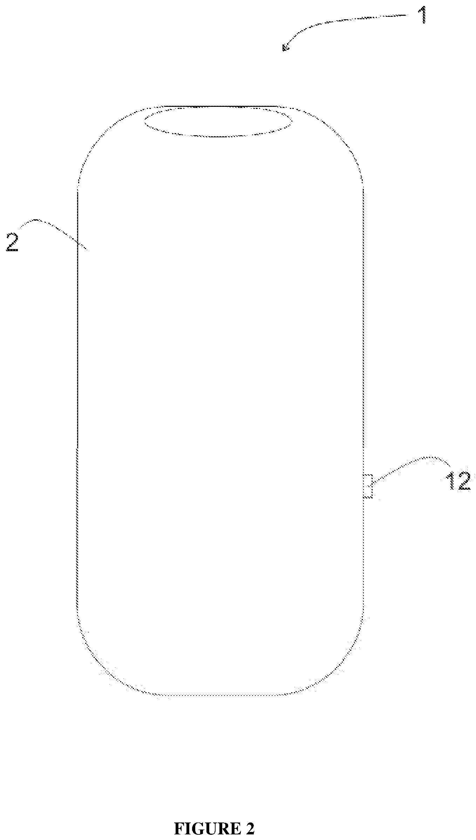

Figure 2 - Outside and side view of the device for cleaning tartar by means of sound wave with the trigger mechanism.

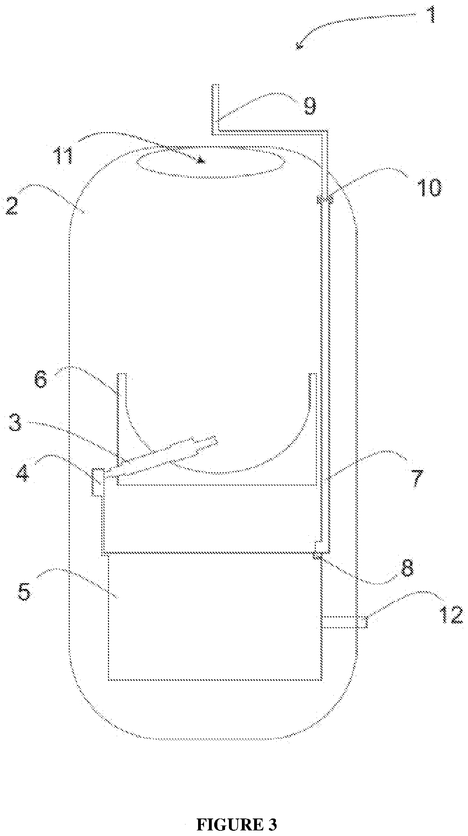

Figure 3 - View of the side internal parts of the device for cleaning tartar by means of sound wave.

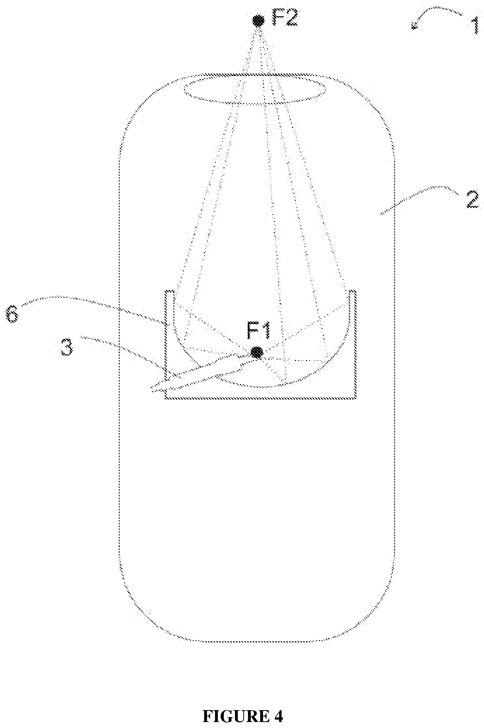

Figure 4 - Schematic representation of the focal angles of the sound wave generator in the device for cleaning tartar by means of sound wave.

The parts in the figures are numbered and their corresponding numbers are listed below.
1. Device for cleaning tartar by means of sound wave
2. Outer cover
3. Generator
4. Socket
5. Control unit
6. Redirector
7. Transfer lever
8. Trigger
9. Focus adjuster tip
10. Slot
11. Sound wave outlet hole
12. Key
F1. Production focus
F2. Secondary focus

### Detailed description of the invention:

The invention relates to a device for cleaning tartar by means of sound wave (1) that disrupts the structure of the tartar by transmitting sound waves to the tartar and allows it to be removed from the mouth. Cleaning the tartar formed in the mouth is important in terms of dental health. Different devices are used by dentists for this purpose.

The device for cleaning tartar by means of sound wave (1) of the invention will mainly include an outer cover (2) designed to be used by physicians with their hands, which includes a slot (10) and a sound wave outlet hole (11), a control unit (5) in this outer cover (2), a generator (3) operating connected to this control unit (5) with socket (4) and generating sound waves, a trigger (8) controlling the time when the generator (3) will operate and a focus adjusting tip (9) determining the distance of the generator to the tartar.

Since the generator (3) to be used in the device for cleaning tartar by means of sound wave (1) of the invention operates with different methods, there will also be a redirector (6) or redirectors (6) focusing the sound waves to be designed differently in these different embodiments of the invention.

The device for cleaning tartar by means of sound wave (1) of the invention will provide the transmission of the physical effect created by these sound waves to the tartar by refocusing the sound waves produced and focused in the device on the tartar. It is aimed to disrupt the structures of the tartar with the resonance formed and to disintegrate and disintegrate the structures they hold in the mouth in this way. Tartar that will be removed from the mouth and the places where they are held, ensuring oral hygiene will make the dental enamel and gums accessible in this way.

The invention will have an outer cover (2) that dentists can use with their hands for application in line with its purpose. The sound waves produced in the device will be guided and the device parts will be protected during the application thanks to this outer cover (2). The physician will be able to direct the device for cleaning tartar by means of sound wave (1) by using their hands at the same time thanks to the outer cover (2), which is expected to be ergonomically designed. It is planned to increase the use of the invention thanks to the work freedom to be obtained in this way. The material to be used in the production of the outer cover (2) is hard, robust, and sterilizable. There will be a slot (10) on the outer cover (2) where the focus adjusting tip (9) can be fixed to the device (1) by passing through. There will also be a sound wave outlet hole (11) in the extension of the outer cover (2) towards the tartar so that the sound waves to be formed by the modules in the outer cover (2) can reach the tartar in the desired way.

There will be a generator (3) in order to perform the tartar cleaning task in accordance with the stated purposes of the invention. The generator (3) will mainly be used for the production of ultrasonic sound waves. Electrohydraulic, electromagnetic, and piezoelectric methods are available in the known state of the art for ultrasonic sound wave generation. It will be possible to use a generator (3) using any of these known techniques in the invention of the device for cleaning tartar by means of sound wave (1).

The ultrasonic sound waves produced by the generator (3), which will be located in the outer cover (2), will benefit from producing outside the body and focusing on the tartar direction. The ultrasonic sound waves produced by the generator (3) are intended to disrupt the structure of the tartar in accordance with the intended use of the invention by focusing on the tartar as desired.

The point where the ultrasonic sound waves created by the generator (3) start to spread will be the production focus (F1). The propagation of ultrasonic sound waves arising from this production focus (F1), which is determined as the point source for easy performance of physical calculations and ensuring the purpose of the invention, may vary depending on the position of the generator (3) and the sound wave production method. The redirector (6) will be used in the device in order for the sound waves produced by the generator (3) to be used in the device for cleaning tartar by means of sound wave (1) will be focused on the tartar. The redirector (6) is basically the structures that direct the sound waves produced by the generator (3) in the production focus (F1) to combine in the secondary focus (F2). In different embodiments of the invention, the redirectors (6) may be conical or in the form of an ellipse or acoustic lens.

The ultrasonic sound waves produced by the generator (3) will be focused on the tartar, and the structural integrity of the tartar will be disrupted and separated from the structures in the mouth where they are held and will be easily removed from the mouth thanks to the redirector (6). The orientation direction of the redirector (6) must be in the direction of the sound wave outlet hole (11) on the outer cover (2). The ultrasonic sound waves produced by the generator (3) will be directed through the outer cover (2) towards the space (11) and will be focused again in the secondary focus (F2) in this way.

In different embodiments of the invention, the generator (3) may not be located in the outer cover (2). The generator (3) will be produced in a main station where the outer cover (2) is connected, and the sound waves will be transferred into the manually designed outer cover (2) by a suitable method in this case. The geometric place in the outer cover (2) where the sound waves are transferred can be defined as the production focus (F1), and the redirector (6) can be used to focus the sound waves in the secondary focus (F2) towards the sound wave outlet hole (11) on the outer cover (2) in this embodiment. In this embodiment of the invention, it is possible to use more than one director (6) for the desired transmission of sound waves between the main station and the outer cover (2).

The basic physical concept required for the operation of the invention is based on increasing the effect of the sound wave bundle, which is produced and has little shock effects on its own, by focusing on a point target. The sound wave makes the air in the environment where it is emitted mobile with itself. The ultrasonic sound waves that spread in the form of sinusoidal waves constitute the situation that accelerates the air the most at the peak points of the wave. Therefore, in acoustic physics, sound waves create compression, and expansion zones in the air at the length of wavelengths. These compressions physically contact the surface and form an impulse in the areas where the sound wave falls on. This impulse, which is formed by the contact of sound waves with the surface, creates vibration on the surface on which they fall and its effects increase with resonance since it includes the physical effect and release steps that occur repeatedly in the wavelength range. The power of the impulse can be increased by providing interference of sound waves coming from different places but with the same wavelength at a focal point. The physical acoustic concepts required for the fulfillment of the basic task of the invention can be explained in the simplest form as the first spreading of the ultrasonic sound waves of the same feature produced by the generator (3) in the production focus (F1) and then focusing on the secondary focus (F2) with the redirector (6) and the impulse formed by the transmission of these shock waves to the tartar provides the structural deterioration of the tartar. This structural degradation can be explained by the pressure-dependent fracture, fragmentation, cavity formation and dynamic fatigue effects defined in the fracture mechanisms of the tartars.

The focus adjuster tip (9) has a protrusion that will physically touch the tartar. Therefore, the focus adjuster tip (9) is designed as a patient-specific consumable. It is aimed to prevent contamination that may occur due to the use of the same focus adjusting tip (9) in different patients in this way. The focus adjusting tip (9) has a diameter that can pass through the slot (10) on the outer cover (2) and is designed to be in the secondary focus (F2) of the device for cleaning tartar by means of sound wave (1) at one end and to be fixed to the transfer arm (7) with a tight fit at the other end. The physical position of the production focus (F1) is fixed since the redirector (6) in the device for cleaning tartar by means of sound wave (1) is in a fixed position. In this case, the position of the secondary focus (F2) is also known in advance. Thanks to the structure of the focus adjusting tip (9) exactly where this secondary focus (F2) is located, when the tip of the focus adjusting tip (9) is contacted with the tartar by the operator, the position required for the device for cleaning tartar by means of sound wave (1) to perform its desired task can be determined by the operator. The axis of the tip of the secondary focus (F2) is planned to be offset from the axis of the point where it is connected to the transfer arm (7) and will pass to the slot (10) on the outer cover (2) in order for the focus adjusting tip (9) to fulfill the specified features. It will be designed to prevent the sound waves produced by the generator (3) in the least way in this way.

The transfer lever (7) is basically a stick-shaped trigger mechanism that carries the mechanical force transmitted by the focus adjuster tip (9) to the trigger (8). The device for cleaning tartar by means of sound wave (1) of the invention will have a transfer arm (7) to provide the specified features. The transfer arm (7) will ensure that the secondary focus (F2), in which the redirector (6) in the outer cover (2) focuses the sound waves, is on the tartar. The transfer lever (7) will be used with the focus adjuster tip (9). The transfer lever (7), which will be connected to the focus adjuster tip (9) at one end, will be connected to the trigger (8) at the other end.

The trigger (8) is in a structure that determines the physical contact applied to it by the transfer arm (7) and transmits this information to the control unit (5) in order for the invention to operate in accordance with the desired purposes. The use of piezoelectric materials or load cells in the determination of physical contact is possible, as well as mechanical triggers in the determination of contact. The trigger (8) assembly operates by transmitting the physical thrust formed when the focus adjuster tip (9) is touching the tartar to the trigger (8) by the transfer lever (7). The change caused by the effect specified in the mechanism is sent to the control unit (5) and it is ensured that the tartar is in the secondary focus (F2) determined by the device for cleaning tartar by means of sound waves (1).

It is also possible to measure the pressure of the focus adjusting tip (9) on the tartar if piezoelectric or load cell is used as the trigger (8). It will be possible to use new features that can be added to the operating features of the device for cleaning tartar by means of sound wave (1) in this case.

The device for cleaning tartar by means of sound wave (1) of the invention will have a key (12) to enable the operator to give permission to work during its use. This key (12) is on the outer cover (2) and can be pressed with the finger of the operator, as well as it can be designed to be pressed with the foot as a pedal in order to provide free movement in different embodiments of the invention. It will be expected that this key (12) will be off as a priority condition for the operation of the device (1) of the invention.

The control unit (5) to be located in order to fulfill the desired features of the invention is basically the unit that provides the controls to determine the operating conditions of the generator (3) to be located in the device (1). The operating timing of the generator (3) in the device (1) of the invention can be adjusted by means of the control unit (5) to be located in the main station or outer cover (2). The control unit (5) to be used to determine the operating timing of the generator (3) in the simplest state of the invention will direct the secondary focus (F2) transmitted to it by the trigger (8) to the socket (4) in such a way that the voltage required for the operation of the generator (3) is transferred to the generator (3) in all cases where the suitability status for the operation to be transmitted by the operator via the key (12) is provided at the same time. The control unit (5) will be in a structure that can communicate with the key (12) and trigger (8), transmit the desired operating conditions to the generator (3) with the help of socket (4) and control the power supply in this context.

The socket (4) will be used to transfer the electrical current to the generator (3) and thus to operate the generator (3). The generator (3) will enable it to operate by generating the sound wave at the desired wavelength at the desired time by transmitting the voltage transmitted to it by the control unit (5) and having the desired electrical properties (voltage, current, etc.) to the generator (3).

The invention may consist of a station and a handheld device for the purposes of this invention. The embodiment of the invention where the generator (3) is located in the technically feasible hand station is shown in the figures. It is possible to use a main station for the production of sound waves and the outer cover (2) as a handheld device for the operator to apply in different embodiments of the invention. It is possible that the parts defined as inside the outer cover (2) are located in the main station for this reason.

It has the potential to increase the working comfort and effectiveness and professional income of the end user since faster, more comfortable and more sheltered treatments will be applied when this device is used.

## Claims

1. A device (1) for cleaning tartar by means of sound wave that is configured to disrupt the physical integrity of the tartar and ensure the removal of the tartar without the need for the use of liquid for cooling purposes by directing sound waves emitted from a production focus (F1) to a secondary focus (F2) located on the tartar, comprising:
- an outer cover (2) including a slot (10) and a sound wave outlet hole (11), said outer cover (2) designed in a way that the dentists can use it with their hands,
- a control unit (5) in the outer cover (2),
- a generator (3) operating connected to the control unit (5) with socket (4) and generating sound waves,
- a trigger (8) controlling the time when the generator (3) will operate, and
- at least one redirector (6) positioned to direct the sound waves emitted from the production focus (F1) to the secondary focus (F2) located on the tartar;
- a key (12) for enabling the operator to give permission to work by pressing on the key;
and **characterized by**
- a detachably designed focus adjuster tip (9) having a protrusion for physically touching the tartar,
wherein the focus adjuster tip (9) can be fixed to the device (1) by passing through the slot (10),
and the control unit (5) enables the generator (3) to operate by transmitting voltage with the necessary electrical properties to the generator (3) with the realization of the following conditions for generating the desired sound wave properties:
- pressing the key (12) and
- transferring physical contact applied to the focus adjuster tip (9) to the trigger (8) by means of a transfer lever (7).

## Patentansprüche

1. Vorrichtung (1) zum Reinigen von Zahnstein mittels Schallwellen, die so konfiguriert ist, dass sie die physikalische Integrität des Zahnsteins unterbricht und die Entfernung des Zahnsteins ohne die Notwendigkeit der Verwendung von Flüssigkeit zu Kühlzwecken sicherstellt, indem Schallwellen, die von einem Erzeugungsfokus (F1) emittiert werden, auf einen Sekundärfokus (F2) gerichtet werden, der sich auf dem Zahnstein befindet, umfassend:
- eine äußere Abdeckung (2) mit einem Schlitz (10) und einem Schallwellenaustrittsloch (11), wobei die äußere Abdeckung (2) so gestaltet ist, dass die Zahnärzte sie mit ihren Händen benutzen können,
- eine Steuereinheit (5) in der äußeren Abdeckung (2),
- einen Generator (3), der über eine Buchse (4) mit der Steuereinheit (5) verbunden ist und Schallwellen erzeugt,
- einen Auslöser (8), der den Zeitpunkt steuert, zu dem der Generator (3) arbeitet, und
- mindestens einen Umlenker (6), der so angeordnet ist, dass er die vom Erzeugungsfokus (F1) ausgesandten Schallwellen zum Sekundärfokus (F2) auf dem Zahnstein lenkt;
- eine Taste (12), mit der der Bediener durch Drücken der Taste die Erlaubnis zum Arbeiten erteilen kann;
und **gekennzeichnet durch**
- eine abnehmbar gestaltete Fokuseinstellspitze (9) mit einem Vorsprung zum physischen Berühren des Zahnsteins,
wobei die Fokuseinstellspitze (9) durch den Schlitz (10) hindurch an der Vorrichtung (1) befestigt werden kann,
und die Steuereinheit (5) den Generator (3) in Betrieb setzt, indem sie eine Spannung mit den erforderlichen elektrischen Eigenschaften an den Generator (3) überträgt, wobei die folgenden Bedingungen zur Erzeugung der gewünschten Schallwelleneigenschaften realisiert werden:
- Drücken der Taste (12) und
- Übertragen des auf die Fokuseinstellspitze (9) ausgeübten physischen Kontakts auf den Auslöser (8) mittels eines Übertragungshebels (7).

## Revendications

1. Un dispositif (1) pour nettoyer le tartre au moyen d'ondes sonores configuré pour perturber l'intégrité physique du tartre et assurer l'élimination du tartre sans nécessiter l'utilisation de liquide à des fins de refroidissement en dirigeant les ondes sonores émises à partir d'un foyer de production (F1) vers un foyer secondaire (F2) situé sur le tartre, comprenant :
- un couvercle extérieur (2) comprenant une fente (10) et un trou de sortie d'ondes sonores (11), ledit couvercle extérieur (2) étant conçu de manière à ce que les dentistes puissent l'utiliser avec leurs mains,
- une unité de contrôle (5) dans le couvercle extérieur (2),
- un générateur (3) fonctionnant relié à l'unité de contrôle (5) par une prise (4) et générant des ondes sonores,
- un déclencheur (8) contrôlant le temps où le générateur (3) fonctionnera, et
- au moins un redirecteur (6) positionné pour diriger les ondes sonores émises par le foyer de production (F1) vers le foyer secondaire (F2) situé sur le tartre ;
- une touche (12) permettant à l'opérateur de donner l'autorisation de travailler en appuyant sur la touche ;
et **caractérisé par**
- un embout de réglage de foyer (9) conçu de manière amovible présentant une protubérance permettant de toucher physiquement le tartre,
dans lequel l'embout de réglage de foyer (9) peut être fixé au dispositif (1) en passant à travers la fente (10),
et l'unité de contrôle (5) permet au générateur (3) de fonctionner en transmettant au générateur (3) une tension ayant les propriétés électriques nécessaires à la réalisation des conditions suivantes pour générer les propriétés d'ondes sonores souhaitées :
- appuyer sur la touche (12) et
- transférer le contact physique appliqué à l'embout de réglage de foyer (9) au déclencheur (8) au moyen d'un levier de transfert (7).
